# FASCICULE DE BREVET EUROPEEN

(11) **EP 0 767 781 B1**
(45) Date de publication et mention de la délivrance du brevet: **31.03.1999**
(21) Numéro de dépôt: 95924375.9
(22) Date de dépôt: 28.06.1995
(51) Int. Cl.: C07C 237/50, C11D 1/62

(54) **NOUVEAUX DERIVES D'AMMONIUMS QUATERNAIRES, LEUR PROCEDE DE PREPARATION ET LEUR UTILISATION COMME AGENTS DE SURFACE**
QUATERNÄRE AMMONIUMDERIVATE, VERFAHREN ZU IHRER HERSTELLUNG UND IHRE VERWENDUNG ALS OBERFLÄCHENAKTIVE VERBINDUNGEN
NOVEL QUATERNARY AMMONIUM DERIVATIVES, PREPARATION METHOD THEREFOR AND USE THEREOF AS SURFACTANTS

(30) Priorité: 28.06.1994 FR 9407930
(43) Date de publication de la demande: 16.04.1997
(73) Titulaire: SOCIETE D'EXPLOITATION DE PRODUITS POUR LES INDUSTRIES CHIMIQUES-SEPPIC, F-75321 Paris Cédex 07 (FR)
(72) Inventeur: BRANCQ, Bernard, F-78150 Le Chesnay (FR); BOITEUX, Jean-Pierre, F-81710 Saix (FR)
(74) Mandataire: Hubert, Philippe
(86) Numéro de dépôt international: FR9500858
(87) Numéro de publication internationale: WO9600719

(56) Documents cités:
- EP-A- 0 302 329
- FR-A- 1 326 561
- FR-A- 1 498 579
- GB-A- 1 114 516
- GB-A- 1 234 408
- CHEMICAL ABSTRACTS, vol. 65, no. 12, 5 Décembre 1966 Columbus, Ohio, US; abstract no. 18744c, colonne 18744; & JP-A-41 006 237 (YOSHIMURA OIL CHEMICAL INDUSTRY CO., LTD) 14 Mai 1962

## Description

La présente invention a pour objet de nouveaux dérivés d'ammoniums quaternaires, leur procédé de préparation et leur utilisation comme agents de surface.

L'invention trouve notamment application dans la préparation de compositions moussantes, nettoyantes, émulsionnantes et conditionnantes destinées à être utilisées dans les domaines pharmaceutique, cosmétique, de l'hygiène, de la détergence, et du textile.

On sait que pour réaliser, à un coût relativement bas, des formules moussantes, nettoyantes, émulsionnantes ou conditionnantes présentant simultanément de bonnes propriétés en terme d'efficacité, de protection et de dermo-compatibilité, il est actuellemment nécessaire de combiner différents agents de surface, ayant chacun une activité et des propriétés particulières.

C'est ainsi que d'une façon générale une formule moussante ou nettoyante comprend aujourd'hui au moins trois types différents d'agents de surface choisis parmi les quatre grandes familles de tensio-actifs que sont les anioniques, les amphotères, les cationiques et les non-ioniques.

Le rôle de ces agents de surface connus peut être schématisé de la manière suivante.

Les agents tensio-actifs anioniques présentent de bonnes propriétés moussantes et constituent généralement le produit nettoyant de base.

A titre d'agents anioniques couramment utilisés, notamment dans les domaines pharmaceutique, cosmétique, de l'hygiène, de la détergence et du textile, on peut citer les alkyl éther sulfates salifiés, les alkylsulfates salifiés, les α-oléfines sulfonates salifiées et les paraffines sulfonates salifiées ainsi que leurs mélanges.

Parmi ceux-ci, le lauryl éther sulfate de sodium est particulièrement préféré.

Les agents tensio-actifs amphotères sont généralement des produits nettoyants permettant d'améliorer la tolérance et la stabilité de la mousse.

A titre d'agents tensio-actifs amphotères couramment utilisés, on citera ici les alkylbétaïnes, les alkylamidobétaïnes, les sultaines, les dérivés d'imidazoline, les phosphobétaïnes, les amphopolyacétates, les amphopropionates ainsi que leurs mélanges.

Parmi ceux-ci, la cocamidopropylbétaïne est particulièrement préférée.

Les agents tensio-actifs non-ioniques présentent des propriétés solubisantes ou émulsionnantes, ou encore pour certains d'entre eux (notamment les alkylpolyglucosides) épaississantes ou moussantes.

A titre d'agents tensio-actifs non ioniques, on citera ici l'huile de ricin hydrogénée, les polysorbates, les alcools gras éthoxylés, les acides gras éthoxylés, les alkylpolyglucosides ou encore les amides de coprah ainsi que leurs mélanges.

Parmi ceux-ci, la diéthanolamide de coprah est particulièrement préférée, notamment en raison de ses propriétés surgraissantes.

Les agents tensio-actifs cationiques présentent un excellent effet "conditionneur", c'est-à-dire qu'ils permettent d'améliorer la souplesse et l'aspect des cheveux en les rendant ainsi plus faciles à déméler ou à coiffer.

Ces produits présentent de faibles propriétés nettoyante ou moussante. En outre, leur utilisation entraîne une diminution de la stabilité de la mousse. C'est la raison pour laquelle ils sont généralement associées à l'utilisation simultannée d'un agent tensio-actif amphotère.

Les agents tensio-actifs cationiques sont notamment utilisés dans les baumes démélants, les shampoings dits "deux en un" et quelquefois pour leurs propriétés émulsionnantes.

A titre d'agents tensio-actifs cationiques couramment utilisés, on peut citer les dérivés d'ammoniums quaternaires, comme en particulier le chlorure de cétyltriammonium (CTAC), ou le chlorure de diméthyldistéarylammonium (DMDSAC).

On peut utiliser alternativement les polymères cationiques.

Il a été découvert, et ceci constitue le fondement de la présente invention, une nouvelle famille de dérivés d'ammoniums quaternaires présentant simultanément les propriétés des agents amphotères, non-ioniques et cationiques et permettant ainsi la réalisation de formules moussantes, nettoyantes, émulsionnantes ou conditionnantes, comportant un nombre limité d'ingrédients. En particulier, il est possible au moyen des nouveaux dérivés d'ammoniums quaternaires conformes à la présente invention de réaliser des formules moussantes binaires combinant un composé de l'invention à un agent anionique connu.

Les composés conformes à la présente invention répondent à la formule suivante : 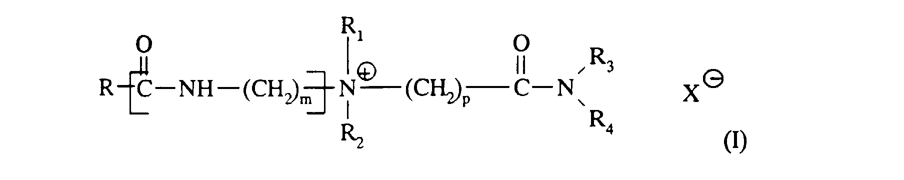 dans laquelle :
R représente un radical alkyle ou alcényle, linéaire ou ramifié, ayant de 5 à 29 atomes de carbone ;
m est un nombre entier égal à 2 ou 3 ;
R1, R2 représentent indépendamment :
   - un radical alkyle ayant de 1 à 4 atomes de carbone ;
   - un radical hydroxyméthyle, hydroxyéthyle ou hydroxypropyle ;
p est un nombre entier compris entre 1 et 3 ;
R₃ représente un groupement : 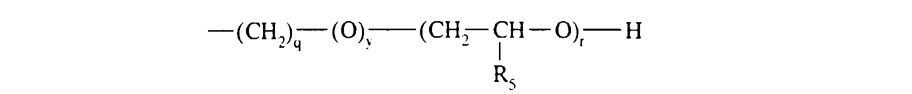 dans lequel :
   · q est un nombre entier compris entre 1 et 5 ;
   · y est un nombre entier égal à 0 ou 1 ;
   · R₅ représente un atome d'hydrogène ou un radical méthyle ;
   · r est un nombre entier compris entre 0 et 10 étant précisé que r et y ne peuvent être simultanément égaux à 0 ;

      - (CH₂)ₛ - (CHOH)ₜ - (CH₂)ᵤ - CH₃

      dans lequel :
      · s est un nombre entier compris entre 0 et 2 ;
      · t est un nombre entier compris entre 0 et 6 ;
      · u est un nombre entier compris entre 0 et 5.

         - (CH₂)ᵥ - (CHOH)_{w} - CH₂OH

         dans lequel :
         · v est un nombre entier égal à 0 ou 1 ;
         · w est un nombre entier compris entre 1 et 6 ;
R₄ représente l'atome d'hydrogène, ou a la même signification que R₃ ;
X représente un contre-ion, de préférence un atome de chlore, de brome ou un groupement hydroxyle.

Dans cette définition, l'expression "R₄ a la même signification que R₃" a été utilisée pour des raisons de simplicité et signifie que R₄ peut représenter les mêmes groupements que ceux représentés par R₃, sans pour autant que R₃ et R₄ soient obligatoirement identiques dans ce cas.

Dans leurs applications, les composés conformes à la présente invention peuvent être utilisés sous forme pure mais également sous forme de mélange.

En effet, ces composés peuvent être préparés à partir d'un acide gras pur en conduisant ainsi à des composés dans lesquels le groupement R représente un radical alkyle ou alcényle défini. Ils peuvent être également préparés à partir d'un mélange d'acides gras dont la longueur de chaîne est variable, en conduisant ainsi à un mélange de composés de formule I qui diffèreront par la nature du radical R.

Des essais réalisés dans le cadre de la présente invention ont montré que les composés de formule I présentent des propriétés comparables à celles des agents de surface amphotères au niveau du pouvoir moussant et épaississant, à celles des agents de surface non ioniques au niveau du pouvoir épaississant et surgraissant, et à celles des agents de surface cationiques au niveau de l'effet conditionneur.

Une sous famille préférée de composés de l'invention est constituée par les composés de formule I dans laquelle :
R représente un radical alkyle ou alcényle, linéaire ou ramifié ayant 7 à 17 atomes de carbone, choisi de préférence dans le groupe comprenant les radicaux
CH₃-(CH₂)₆- ; CH₃-(CH₂)₈- ; CH₃ (CH₂)₁₀- ; CH₃(CH₂)₁₂- ; CH₃(CH₂)₁₄-; CH₃-(CH₂)₁₆- ; CH₃-(CH₂)₇ - CH = CH- (CH₂)₇ - ; CH₃-(CH₂)₅- CH = CH- (CH₂)₇ - ; CH₃- (CH₂)₅-CHOH - CH₂ - CH = CH - (CH₂)₇ ;CH₃- (CH₂)₄ - CH = CH - CH₂ - CH = CH-(CH₂)₇- ; CH₃-(CH₂-CH=CH)₃- (CH₂)₇- ; CH₂ = CH-(CH₂)₈ ;
m, R₁, R₂, p, R₃ et R₄ étant tels que définis précédemment.

Parmi ceux-ci, les composés particulièrement préférés sont ceux de formule I dans laquelle R₃ représente un radical choisi dans le groupe constitué par les radicaux hydroxy-2-éthyle, hydroxy-2-propyle, hydroxy-3-propyle, méthyle et pentahydroxy-2,3,4,5,6 hexyle.

Les composés actuellement les plus préférés, dans le cadre de la présente invention sont les composés de formule I dans laquelle :
m est égal à 3 ;
R₁ et R₂ représentent un radical méthyle ;
p est égal à 1 ;
R₃ représente un radical hydroxy-2-éthyle ;
R₄ représente l'atome d'hydrogène ou un radical hydroxy-2-éthyle ;
X représente un atome de chlore ou un groupement hydroxyle ; et
R est tel que défini précédemment.

Ces composés répondent donc à la formule :

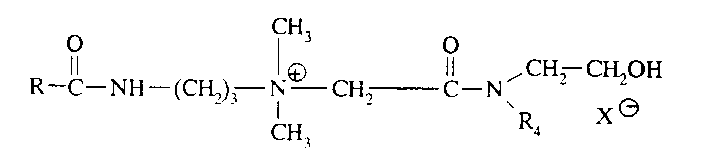

Selon un deuxième aspect, la présente demande vise à couvrir un procédé de préparation des dérivés d'ammoniums quaternaires de formule I précitée.

D'une façon générale ce procédé comprend :
l'amidification d'une amine de formule : 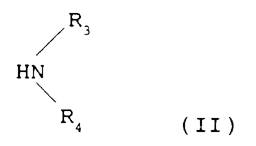 par un composé de formule : 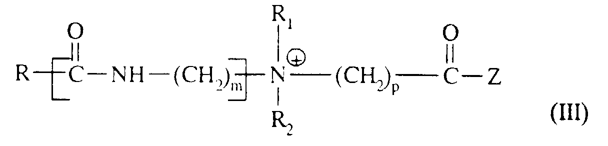 dans laquelle R, m, R₁, R₂, p sont tels que définis précédemment ; et
Z représente un groupement OH, OCH₃, OC₂H₅, OC(O)CH₂Cl, Cl, OM où M représente un métal alcalin.

Cette étape sera désignée dans les exemples par "Etape C".

Selon une caractéristique particulière, le composé de formule (III) précité est obtenu par quaternisation d'une amine de formule : 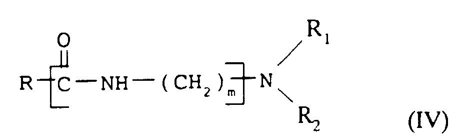 dans laquelle R, m, R₁,et R₂, sont tels que définis précédemment, par un composé de formule :

Cl-CH₂-CO-Z (V)

dans laquelle Z est tel que défini précédemment.

Cette étape sera désignée dans les exemples par "Etape B".

Selon une autre caractéristique particulière, le composé de formule IV est obtenu par condensation d'une aminoalkylamine de formule : 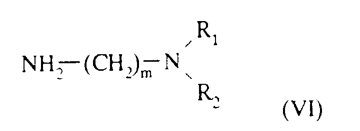 dans laquelle m, R₁, R₂ sont tels que définis précédemment,
sur un acide gras de formule RCOOH ou l'un de ses précurseurs tels que son acide méthylique ou l'un de ses glycérides ; R étant tel que défini précédemment.

Cette étape sera désignée dans les exemples par "Etape A".

Les réactions d'amidification, de quaternisation et de condensation précitées sont des réactions classiques et l'homme de métier pourra se reporter à la littérature existant pour déterminer les conditions appropriées (température, durée, nature des solvants, etc.) pour mener à bien ces réactions.

Il a été constaté, de façon tout à fait inattendue et ceci constitue une originalité du procédé de la présente invention que la couleur des produits de formule (I) peut être considérablement améliorée lorsque l'amidification de l'amine de formule (Il) par le composé de formule (III), est suivie d'un traitement par un composé choisi dans le groupe comprenant l'anhydride acétique et le chlorure de l'acide acétique, de façon à réduire la quantité d'amine résiduelle à une valeur inférieure à 2 %, de préférence inférieure à 1 %.

Les conditions de ce traitement pourront être facilement déterminées par l'homme de métier.

Selon un troisième aspect, la présente demande vise à couvrir des compositions moussantes, nettoyantes, émulsionnantes ou conditionnantes, comprenant au moins un composé de formule (I) tel que défini précédemment, en combinaison avec un agent de surface anionique, dans un rapport pondéral compris entre 99 : 1 et 1 : 99.

Cet agent de surface anionique peut être l'un des agents couramment utilisés mentionnés dans l'introduction de la présente demande. De préférence, il s'agira du lauryl éther sulfate de sodium.

Enfin, selon un quatrième aspect, la présente demande vise à couvrir l'utilisation des dérivés d'ammoniums quaternaires de formule (I) précités pour la préparation de compositions moussantes, émulsionnantes, nettoyantes et conditionnantes.

Les exemples suivants donnés à titre non limitatif illustreront la présente invention.

Dans ces exemples, sauf indication contraire, les pourcentages sont exprimés en poids.

### EXEMPLE 1 : Exemple de préparation d'un composé selon l'invention.

### ETAPE A :

Dans une cuve polyvalente, on ajoute 24,2 kg de diméthylaminopropylamine à 45 kg d'acide gras.

Cet acide gras est en fait un mélange d'acides en C₁₄, C₁₆ et C₁₈ de composition :

| | |
|---|---|
| C₁₄ | 2 % |
| C₁₆ | 45 % |
| C₁₈ | 53 % |

Le mélange ainsi obtenu est chauffé à 155°C jusqu'à obtention d'un indice d'acide inférieur à 5.

L'excès de diméthylaminopropylamine est ensuite éliminé sous pression réduite pour conduire à 58,3 kg d'amide.

### ETAPE B

24,7 kg de l'amide obtenus à l'étape A sont dissous dans 55 kg d'alcool isopropylique.

Ce mélange est porté à 60°C et on y ajoute sous agitation 9,2 kg de monochloroacétate de sodium.

Après 11 heures d'agitation à 100°C, l'ensemble est refroidi à 60°C et filtré.

### ETAPE C

48,5 kg du filtrat obtenu à l'étape B sont chauffés sous pression réduite jusqu'à élimination de l'alcool isopropylique.

On ajoute 3,3 kg de monoéthanolamine au produit ainsi obtenu.

Après 6 heures de réaction à 155°C, on obtient 22 kg d'un solide de couleur beige foncé.

Le produit ainsi obtenu présente les caractéristiques suivantes :

| | |
|---|---|
| Couleur (produit fondu) | 11 Vcs |
| Monoéthanolamine libre | 0,25 % |
| pH (10 %) | 8,32 |

Ce produit présente la formule développée suivante :

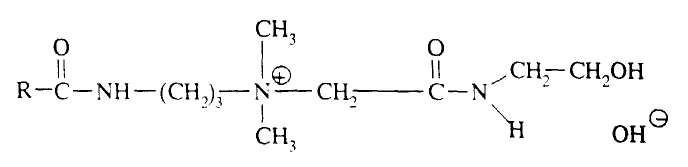

### EXEMPLE 2

### ETAPE A

L'étape A de l'exemple 1 est répétée en remplaçant l'acide gras par un mélange présentant la composition suivante :

| | |
|---|---|
| C₁₀ | 1 % |
| C₁₂ | 55 % |
| C₁₄ | 23 % |
| C₁₆ | 10 % |
| C₁₈ | 2 % |
| C_{18'} | 8 % |

### ETAPE B

22,5 kg d'alkylamidopropyldiméthylamine préparés à l'étape A sont dissous à 50°C dans 37 kg d'alcool isopropylique.

Après addition de 9,5 kg de monocloroacétate de sodium, le mélange est chauffé sous agitation à 95°C pendant 15 heures puis refroidi à 50°C et filtré.

### ETAPE C

46,1 kg du filtrat obtenu à l'étape B sont chauffés sous pression réduite jusqu'à élimination de l'alcool isopropylique.

Après addition de 3,0 kg de monoéthanolamine, le milieu réactionnel est porté sous agitation à 170°C et maintenu à cette température pendant 6 heures.

Le mélange réactionnel ainsi obtenu est dilué à environ 60°C dans l'eau pour conduire à 32,7 kg d'un liquide limpide comportant 39,8 % d'extrait sec et présentant les caractéristiques suivantes :

| | |
|---|---|
| Couleur | 10 Vcs |
| Monoéthanolamine libre | 0,13 % |
| pH (produit) | 7,55 |
| Viscosité à 20°C | 1250 mPa.s |

### EXEMPLE 3

### ETAPE B

25,4 kg d'alkylamidopropyldiméthylamine préparés à l'étape A de l'exemple 2 et 3,9 kg d'alcool méthylique sont chaufffés à 80°C sous agitation.

9,8 kg de monochloroacétate de méthyle sont ajoutés en 1 heure, et le mélange réactionnel ainsi obtenu est maintenu à 80°C pendant 6 heures.

### ETAPE C

32 kg du mélange réactionnel obtenu à l'étape B sont portés à 60°C et additionnés en une demi-heure sur un mélange chauffé à 90°C de 4 kg de monoéthanolamine et de 0,05 kg de méthylate de sodium en solution à 30 % dans de l'alcool méthylique.

Après 5 heures de réaction sous vide à 100°C, le produit obtenu est refroidi et dilué à 41,3 % d'extrait sec.

Le liquide limpide ainsi obtenu présente les caractéristiques suivantes :

| | |
|---|---|
| Couleur | 5 Vcs |
| Monoéthanolamine libre | 0,9 % |
| pH (5 %) | 5,9 |
| Viscosité à 20°C | 51 mPa.s |

### EXEMPLE 4

Influence de l'anhydride acétique sur la couleur du produit fini.

Les étapes A et B de l'exemple 1 sont reproduites afin de préparer 62,5 kg de filtrat.

### ETAPE C

62,5 kg du filtrat ainsi préparé sont chauffés sous pression réduite jusqu'à élimination totale de l'alcool isopropylique.

On ajoute 3,5 kg de monoéthanolamine au produit ainsi obtenu.

Après 3 heures de réaction à 150°C le milieu réactionnel est refroidi à 70°C et 0,6 kg d'anhydride acétique sont additionnés.

Après 3 heures d'agitation à 70°C, on obtient 26,5 kg d'un solide de couleur beige clair.

Le produit obtenu présente les caractéristiques suivantes :

| | |
|---|---|
| Couleur (produit fondu) | 8 Vcs |
| Monoéthanolamine libre | 0,32 % |
| pH (10 %) | 7,75 |

Cet exemple démontre l'influence de l'anhydride acétique sur la couleur du produit fini.

En effet la couleur du produit préparé à l'exemple 1 est de 11 Vcs.

Le produit préparé dans les mêmes conditions avec ajout d'anhydride acétique au cours de l'étape C présente une couleur de 8 Vcs, assez nettement améliorée.

### EXEMPLE 5

Influence de l'anhydride acétique sur la couleur du produit fini.

Les étapes A et B de l'exemple 2 sont reproduites afin de préparer 52,0 kg de filtrat.

### ETAPE C

52,0 kg de filtrat ainsi obtenu sont chauffés sous pression réduite jusqu'à élimination totale de l'alcool isopropylique.

Après addition de 3,2 kg de monoéthanolamine, le milieu réactionnel est agité à 180°C pendant 3 heures, puis refroidi à 70°C et enfin additionné de 1,5 kg d'anhydride acétique.

Après 3 heures de réaction, le produit brut ainsi obtenu est dilué dans l'eau pour conduire à 50 kg d'un liquide comprenant 39 % d'extrait sec et présentant les caractéristiques suivantes :

| | |
|---|---|
| Couleur | 3 Vcs |
| Monoéthanolamine libre | 0,34 % |
| pH (produit) | 6,63 |
| Viscosité à 20°C | 1670 mPa.s |

Cet exemple démontre l'influence de l'anhydride acétique sur la couleur du produit fini.

En effet la couleur du produit préparé à l'exemple 2 est de 10 Vcs.

Le produit préparé dans les mêmes conditions avec ajout d'anhydride acétique au cours de l'étape C présente une couleur de 3 Vcs, nettement améliorée.

### Mise en évidence des propriétés des composés selon l'invention :

Les propriétés des composés selon l'invention ont été comparées à celles des agents de surface anionique, amphotère, non ionique et cationique couramment utilisés.

A cet effet, les méthodes de détermination suivantes ont été utilisées :

| **PROPRIETES** | **METHODES** |
|---|---|
| Volume mousse | ISO 696-1975 |
| Pouvoir mouillant | AFNOR NFT 73-406 |
| Pouvoir solubilisant MIP | Doc technique PROTEOL K 25 réf. GL/0239/GB/01/January 93 |
| Pouvoir démêlant | KAMATH.Y.K., WIGMANN H.D. Measurement of Combing Forces- J. Soc. Cosmet. Chem., 37, 3, 111-124, 1986 |
| Indice de substanticité vis-à-vis de la kératine | LUTZ D., HOLTZINGER G., KHAIAT A., Substantivity of Cationics to the Hair. A New Determination-Methodology-Cosmet. Toilet. Manufacture, 130-137, 1991/1992 |
| HET-CAM | INVITTOX-HET CAM TEST-INVITTOX PROTOCOL N°47, Janvier 1992 |
| Effet conditionneur | Doc technique PROTEOL VS 22 réf. GL/0113/GB/01/February 92 |

Les résultats obtenus ont été résumés dans le tableau 1.

Ces résultats font apparaître que les composés selon l'invention présentent des propriétés comparables à celles des agents de surface amphotère, non ionique et cationique.

Pour confirmer l'intérêt des composés selon l'invention dans la préparation de compositions moussantes et émulsionnantes, les caractéristiques d'une formule binaire incorporant un composé selon l'invention et un agent de surface anionique ont été comparés aux quatre compositions suivantes :

### Composition A :

Composition binaire incorporant un agent de surface anionique et un agent de surface amphotère.

### Composition B :

Formulation ternaire incorporant un agent de surface anionique, un agent de surface amphotère et un agent de surface non ionique.

### Compositions C et D :

Formulation à quatre composants incorporant un agent de surface anionique, un agent de surface non ionique, un agent de surface amphotère et un agent de surface cationique.

Les résultats obtenus ont été reportés dans le tableau 2.

Ces résultats montrent que la formule binaire incorporant un composé selon l'invention présente des propriétés comparables, voir mêmes supérieures à celles des formulations à quatre composants telles que celles existant aujourd'hui, et très nettement supérieures à celles des formulations à deux ou trois composants.

La composition A présente un bon effet nettoyant, mais aucune des propriétés cosmétiques classiques.

La composition B présente, en raison de la présence d'un agent de surface non ionique, un effet surgraissant qui améliore notamment le toucher de la mousse.

La composition C à quatre composants présente en outre un bon effet conditionneur.

La composition D à quatre composants présente de bonnes propriétés, mais souffre de l'inconvénient de présenter un aspect trouble.

Dans les tableaux 1 et 2 les abréviations suivantes ont été utilisées :

| | |
|---|---|
| LESNA | Lauryl éther sulfate de sodium |
| LSNA | Lauryl sulfate de sodium |
| ma | Matière active |
| SAS | Paraffine sulfonate |
| LAS | Dodécyl benzènesulfonate |
| CAPB | Cocoamidopropylbétaïne |
| DMDSAC | Chlorure de diméthyldistéarylammonium |
| pHi | pH initial |
| MIP | Myristate d'isopropyle |
| es | Extrait sec |
| CTAC | Chlorure de cétyltriammonium. |

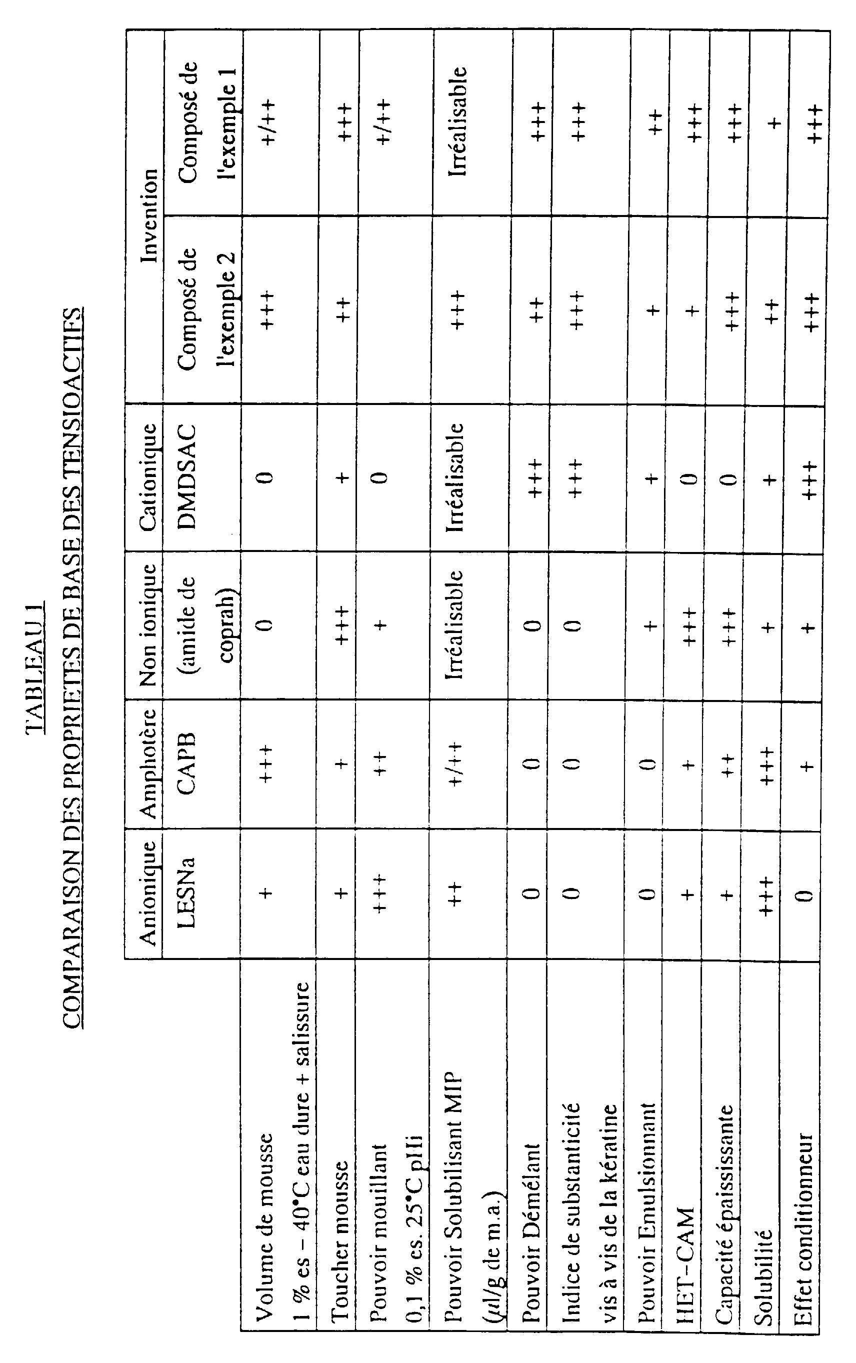

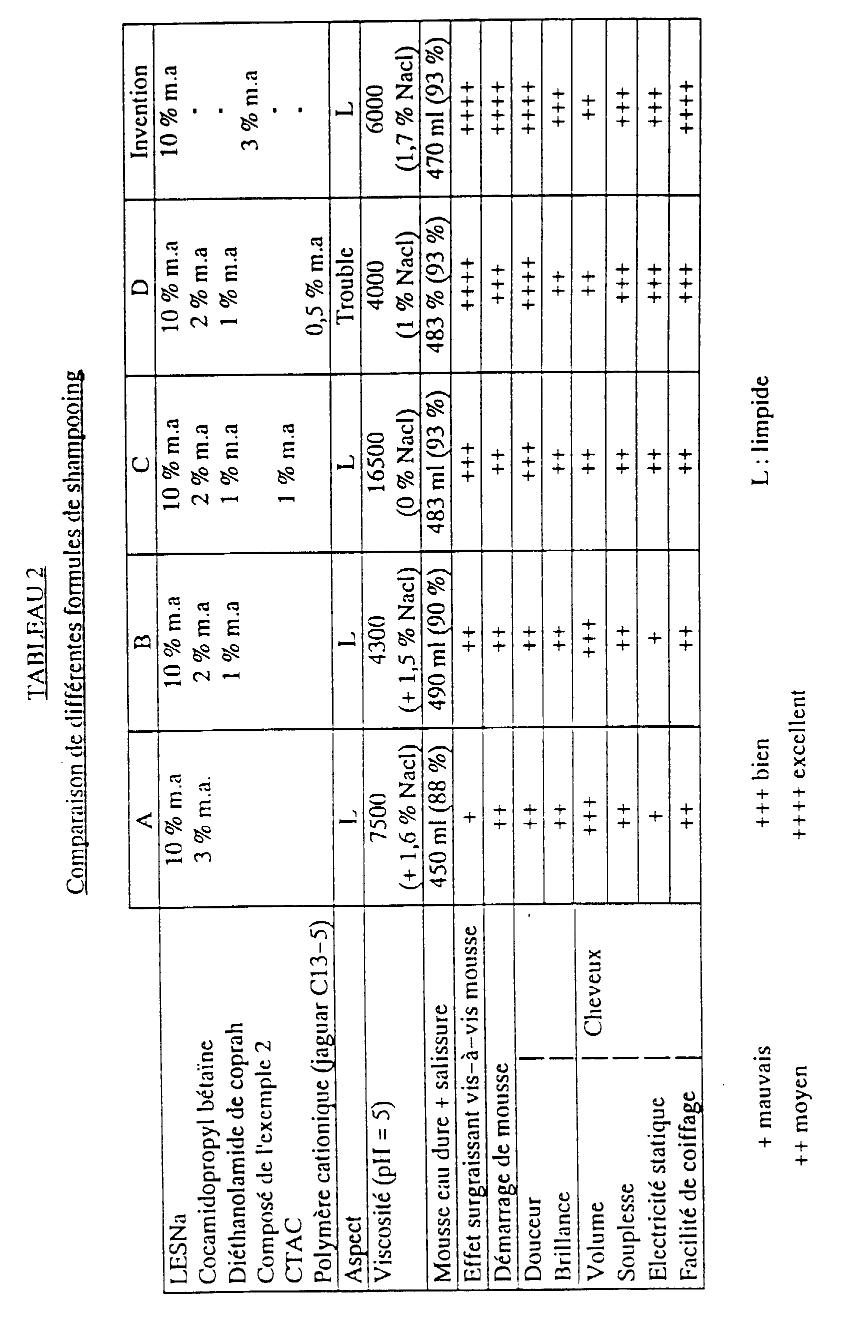

L'effet épaississant des composés de l'invention et en particulier du composé de l'exemple 1 apparaît à dose faible (de l'ordre de 1%). Cet effet est éventuellement modulable par ajout de NaCl et indépendant du pH.

Des essais complémentaires ont montré que les composés conformes à l'invention présentent des propriétés anti-irritantes vis-à-vis des agents de surface anioniques classiquement utilisés, sans aucune altération du pouvoir moussant.

Ainsi, utilisé à 1,5 %, le composé de l'exemple 1 fait chuter l'indice d'irritation du LESNa qui passe de la classe "irritant" à la classe "modérément irritant", tout en améliorant le pouvoir moussant de ce dernier, en procurant à la mousse obtenue richesse et onctuosité.

Les composés conformes à l'invention présentent donc un avantage vis-à-vis des composés non ioniques éthoxylés généralement utilisés pour réduire les propriétés irritantes des agents de surface anioniques, dans la mesure où ces composés non ioniques entraînent une altération du pouvoir moussant.

Il est encore à noter que certains des composés de l'invention, comme en particulier le produit de l'exemple 2, présentent un pouvoir antimicrobien tout à fait intéressant.

C'est ainsi que le produit de l'exemple 2 présente un large spectre d'activité spécifique d'un effet bactériostatique mais également fongistatique.

Cette propriété peut être mise à profit dans divers types de formules telles que gels antimicrobiens, savons liquides antiseptiques et d'une façon générale toutes les formules où l'on cherche à réduire la teneur en conservateur.

Il a enfin été démontré que les composés conformes à la présente invention présentent des propriétés émulsionnantes intéressantes notamment pour la formulation de baumes capillaires ou d'émulsions classiques. Les composés de l'invention jouent, dans ce cas, à la fois le rôle d'actif démêlant et d'émulsionnant.

En raison de la polyvalence de leurs propriétés, les composés de l'invention peuvent être utilisés dans un très grand nombre de formulations.

On donnera ci-après quelques exemples de formulations incorporant les composés conformes à la présente invention.

Ces formulations trouveront notamment application dans le domaine cosmétique, pharmaceutique (savon liquide anti-septique), de l'hygiène ou encore dans le domaine de la détergence (produit à usage ménager ou industriel).

| **Shampooing doux bébé** | |
|---|---|
| LESNA 2.2OE | 6 % ma |
| COMPOSE DE L'EXEMPLE 2 | 1,5 % ma |
| Conservateur | qs |
| Parfum | qs |
| Eau | QSP 100 |

Ce shampoing ne pique pas les yeux et n'emmêle pas les cheveux

| **Shampooing conditionneur pour cheveux abimés :** | |
|---|---|
| LESNA 2.2OE | 9 % ma |
| COMPOSE DE L'EXEMPLE 3 | 3 % ma |
| Conservateur | qs. |
| Parfum | qs |
| Eau | QSP 100 |
| NaCl | qs visco |

| **Crème démélante :** | |
|---|---|
| COMPOSE DE L'EXEMPLE 1 | 2 % ma |
| MONTANOV 68® | 1 % ma |
| Dimethicone | 2 % |
| Conservateur | qs. |
| Parfum | qs. |
| Eau | QSP 100 |

| **Soin lissant pointes sèches :** | |
|---|---|
| COMPOSE DE L'EXEMPLE 1 | 2 % ma |
| Cyclomethicone | 5 % |
| ACRYSOL 44 ® | 2 % |
| Conservateur | qs. |
| Parfum | qs. |
| Eau | QSP 100 |

| **Savon liquide nacré :** | |
|---|---|
| LESNA 2.2OE | 7 % ma |
| COMPOSE DE L'EXEMPLE 2 | 2 % ma |
| Stéarate de glycol | 2 % |
| Conservateur | qs. |
| Parfum | qs. |
| Eau | QSP 100 |
| NaCl | qs visco |

| **Shampooing 2 en 1 :** | |
|---|---|
| LESNA 2.2OE | 12 % ma |
| COMPOSE DE L'EXEMPLE 2 | 2 % ma |
| COMPOSE DE L'EXEMPLE 1 | 0,5 % ma |
| Stéarate de glycol | 2 % |
| Dimethicone 50cps | 1 % |
| Conservateur | qs. |
| Parfum | qs. |
| Eau | QSP 100 |
| NaCI | qs visco |

| **Produit pour permanente (Lotion):** | |
|---|---|
| COMPOSE DE L'EXEMPLE 2 | 1 % ma |
| COMPOSE DE L'EXEMPLE 1 | 2 % ma |
| Thioglycolate d'ammonium | 8 % |
| Soude caustique à 70 % | 10 % |
| Ammoniaque à 28 % | 3 % |
| Epaississant | 1 % |
| Eau | QSP 100 |

| **Gel douche :** | |
|---|---|
| LESNA 2.2OE | 13 % ma |
| COMPOSE DE L'EXEMPLE 3 | 2 % ma |
| Conservateur | qs. |
| Parfum | qs. |
| Eau | QSP 100 |
| NaCl | qs visco |

| **Gel douche 2 en 1 :** | |
|---|---|
| LANOL 84D® | 2 % |
| COMPOSE DE L'EXEMPLE 1 | 2 % ma |
| LESNA 2.20E | 15 %ma |
| Agent opacifiant | 0,5 % |
| Conservateur | qs. |
| Parfum | qs. |
| Eau | QSP 100 |
| NaCI | qs visco |

| **Savon liquide antiseptique :** | |
|---|---|
| COMPOSE DE L'EXEMPLE 2 | 8 % |
| Digluconate de chlorhexidine (solution à 20 %) | 2,5 % |
| Parfum | qs. |
| Eau | QSP 100 |

Il est à noter que dans cette formulation, le composé de l'invention n'est pas associé à un agent de surface anionique. De façon inattendue, l'activité du digluconate de chlorhexidine (qui présente un caractère cationique) n'est pas altérée malgré l'absence d'agent de surface anionique.

| **Crème pour les mains :** | |
|---|---|
| MONTANOV 68 ® | 5 % |
| COMPOSE DE L'EXEMPLE 1 | 0,5 % ma |
| Huile de parrafinc | 15 % |
| Glycérine | 5 % |
| Eau | QSP 100 |
| Parfum | qs. |
| Conservateur | qs. |

| **Lait adoucissant corporel :** | |
|---|---|
| MONTANOV 94 ® | |
| COMPOSE DE L'EXEMPLE 1 | 2 % ma |
| LANOL 2681 ® | 10 % |
| Eau | QSP 100 |
| SEPIGEL 305 ® | 0,8 % |
| Parfum | qs. |
| Conservateur | QS. |

| **Bain moussant hydratant :** | |
|---|---|
| LESNA 2.20E | 15 % ma |
| LSNA | 5 % ma |
| COMPOSE DE L'EXEMPLE 2 | 5 % |
| Stéarate de glycol | 2 % |
| Eau | QSP 100 |
| Parfum | qs. |
| Conservateur | qs. |
| NaCI | qs visco |

| **Assouplissant textile (usage ménager) :** | |
|---|---|
| COMPOSE DE L'EXEMPLE 1 | 4 % |
| Cire de LANOL CT0 ® | 1 % |
| Conservateur | qs. |
| Parfum | qs. |
| Eau | QSP 100 |
| Acide citrique | qs pH 3 |

| **Adoucissant textile (usage industriel) :** | |
|---|---|
| COMPOSE DE L'EXEMPLE 1 | 3 % |
| MONTANOV 68 ® | 0,5 % |
| Eau | QSP 100 |

| **Liquide vaisselle :** | |
|---|---|
| LESNA 2.2OE | 15 % ma |
| SAS ou LAS | 15 % ma |
| COMPOSE DE L'EXEMPLE 2 | 3 % ma |
| Parfum | qs. |
| Conservateur | qs. |
| Eau | QSP 100 |

| **Liquide vaisselle (pour peaux sensibles) :** | |
|---|---|
| LESNA 2.2OE | 20 % ma |
| COMPOSE DE L'EXEMPLE 2 | 5 % ma |
| Parfum | qs. |
| Conservateur | qs. |
| Eau | QSP 100 |

| **Gel détartrant WC :** | |
|---|---|
| COMPOSE DE L'EXEMPLE 2 | 2 % |
| ACRYSOL 44 ® | 5 % |
| Acide acétique | 5 % |
| Colorant | qs. |
| Parfum | qs. |
| Eau | QSP 100 |
| Acide citrique | qs pH 3 |

Dans ces formulations, les produits cités ont leurs dénominations commerciales correspondant aux produits suivants :

| | |
|---|---|
| MONTANOV 68 ® | Cetearyl Glucoside |
| ACRYSOL 44 ® | Urethane/C1-20 Alkyl PEG Copolymer |
| LANOL 84 D ® | Dioctyl Malate |
| LANOL 2681 ® | Coco Caprylate/Caprate |
| SEPIGEL 305 ® | Polyacrylamide et C12-14 Isoparaffin et Laureth-7 |
| Cire de Lanol CTO ® | Cetearyl alcohol et Ceteareth 33 |

## Revendications

1. Dérivés d'ammoniums quaternaires, notamment utiles comme agents de surface cationiques, caractérisés en ce qu'ils répondent à la formule : 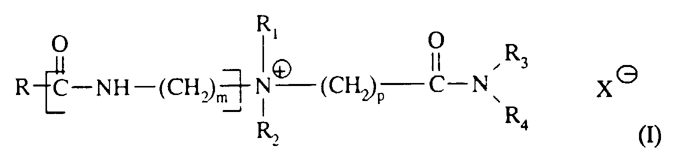 dans laquelle :
R représente un radical alkyle ou alcényle, linéaire ou ramifié, ayant de 5 à 29 atomes de carbone ;
m est un nombre entier égal à 2 ou 3 ;
R1, R2 représentent indépendamment :
- un radical alkyle ayant de 1 à 4 atomes de carbone ;
- un radical hydroxyméthyle, hydroxyéthyle ou hydroxypropyle ;
p est un nombre entier compris entre 1 et 3 ;
R3 représente un groupement : 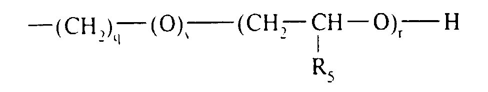 dans lequel :
- q est un nombre entier compris entre 1 et 5 ;
- y est un nombre entier égal à 0 ou 1 ;
- R₅ représente un atome d'hydrogène ou un radical méthyle ;
- r est un nombre entier compris entre 0 et 10 étant précisé que r et y ne peuvent être simultanément égaux à 0 ;
- (CH₂)ₛ - (CHOH)ₜ - (CH₂)ᵤ - CH₃
dans lequel :
- s est un nombre entier compris entre 0 et 2 ;
- t est un nombre entier compris entre 0 et 6 ;
- u est un nombre entier compris entre 0 et 5.
- (CH₂)ᵥ - (CHOH)_{w} - CH₂OH
dans lequel :
- v est un nombre entier égal à 0 ou 1 ;
- w est un nombre entier compris entre 1 et 6 ;
R4 représente l'atome d'hydrogène, ou a la même signification que R3 ;
X représente un contre-ion, de préférence un atome de chlore, de brome ou un groupement hydroxyle.

2. Dérivés d'ammoniums quaternaires de formule I selon la revendication 1, dans laquelle :
R représente un radical alkyle ou alcényle, linéaire ou ramifié ayant 7 à 17 atomes de carbone, choisi de préférence dans le groupe comprenant les radicaux
CH₃-(CH₂)₆- ; CH₃-(CH₂)₈- ; CH₃ (CH₂)₁₀- ; CH₃(CH₂)₁₂- ; CH₃(CH₂)₁₄-; CH₃-(CH₂)₁₆- ; CH₃-(CH₂)₇ - CH = CH- (CH₂)₇ - ; CH₃-(CH₂)₅- CH = CH- (CH₂)₇ - ; CH₃- (CH₂)₅-CHOH - CH₂ - CH = CH - (CH₂)₇ ;CH₃- (CH₂)₄ - CH = CH - CH₂ - CH = CH-(CH₂)₇- ; CH₃-(CH₂-CH=CH)₃- (CH₂)₇- ; H₂C = CH - (CH₂)₈ ;
m, R₁, R₂, p, R₃ et R₄ étant tels que définis à la revendication 1.

3. Dérivés d'ammoniums quaternaires de formule I selon la revendication 2, dans laquelle R₃ représente un radical choisi dans le groupe constitué par les radicaux hydroxy-2-éthyle, hydroxy-2-propyle, hydroxy-3-propyle, méthyle et pentahydroxy-2,3,4,5,6 hexyle.

4. Dérivés d'ammoniums quaternaires de formule 1 selon la revendication 1 dans laquelle :
m est égal à 3 ;
R₁ et R₂ représentent un radical méthyle ;
p est égal à 1 ;
R₃ représente un radical hydroxy-2-éthyle ;
R₄ représente l'atome d'hydrogène ;
X représente un atome de chlore ou un groupement hydroxyle ; et
R est tel que défini à la revendication 1.

5. Dérivés d'ammoniums quaternaires de formule 1 selon la revendication 1 dans laquelle :
m est égal à 3 ;
R₁ et R₂ représentent un radical méthyle ;
p est égal à 1 ;
R₃ représente un radical hydroxy-2-éthyle ;
R₄ représente un radical hydroxy-2-éthyle ;
X représente un atome de chlore ou un groupement hydroxyle ; et
R est tel que défini à la revendication 1.

6. Procédé de préparation des dérivés d'ammoniums quaternaires de formule I selon l'une quelconque des revendications 1 à 5, caractérisé en ce qu'il comprend l'amidification d'une amine de formule : 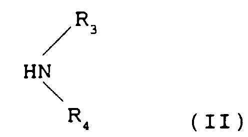 par un composé de formule : 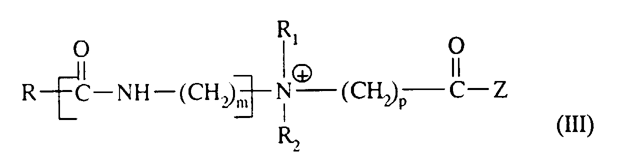 dans laquelle R, m, R₁, R₂, p ont la même signification qu'à la revendication 1 et Z représente un groupement OH, OM, OCH₃, OC₂H₅, OC(O)CH₂Cl, Cl où M représente un métal alcalin.

7. Procédé selon la revendication 6, caractérisé en ce que le composé de formule (III) précitée est obtenu par quaternisation d'une amine de formule : 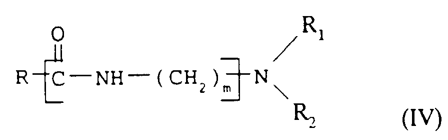 dans laquelle R, m, R₁ et R₂ sont tels que définis à la revendication 1,
par un composé de formule :
Cl-CH₂-CO-Z (V)
dans laquelle Z a la même signification qu'indiqué à la revendication 9.

8. Procédé selon la revendication 6 ou 7, caractérisé en ce que le composé de formule (IV) est obtenu par condensation d'une aminoalkylamine de formule : 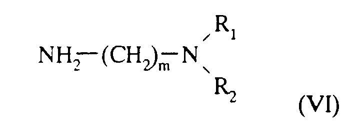 dans laquelle m, R₁, R₂ sont tels que définis à la revendication 1,
sur un acide gras de formule RCOOH ou l'un de ses précurseurs tels que son acide méthylique ou l'un de ses glycérides ; R étant tel que défini à la revendication 1.

9. Procédé selon l'une quelconque des revendications 6 à 8, caractérisé en ce que en vue d'améliorer la couleur du produit de formule (I) précité, l'amidification de l'amine de formule (II) par le dérivé d'ammonium quaternaire de formule (III) est suivie d'un traitement par un composé choisi dans le groupe comprenant l'anhydride acétique et le chlorure de l'acide acétique, de façon à réduire la quantité d'amine résiduelle à une valeur inférieure à 2 %, de préférence inférieure à 1 %.

10. Compositions moussantes, nettoyantes, émulsionnantes ou conditionnantes, caractérisées en ce qu'elles comprennent au moins un composé de formule (I) tel que défini à l'une quelconque des revendications 1 à 5, en combinaison avec un agent de surface anionique, dans un rapport pondéral compris entre 99 : 1 et 1 : 99.

11. Utilisation des dérivés d'ammoniums quaternaires selon l'une quelconque des revendications 1 à 5, pour la préparation de compositions moussantes, nettoyantes, émulsionnantes ou conditionnantes.

## Patentansprüche

1. Quaternäre Ammonium-Derivate, die insbesondere als kationische Tenside (oberflächenaktive Agentien) verwendbar sind, dadurch gekennzeichnet, daß sie der Formel entsprechen: 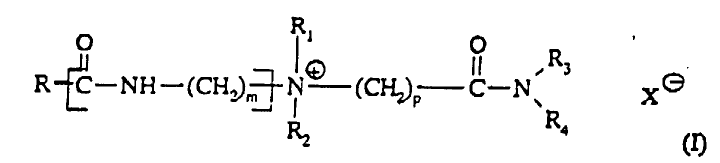 worin bedeuten:
R einen linearen oder verzweigten Alkyl- oder Alkenylrest mit 5 bis 29 Kohlenstoffatomen;
m die ganze Zahl 2 oder 3;
R₁, R₂ unabhängig voneinander:
- einen Alkylrest mit 1 bis 4 Kohlenstoffatomen;
- einen Hydroxymethyl-, Hydroxyethyl- oder Hydroxypropylrest;
p eine ganze Zahl zwischen 1 und 3;
R₃ eine Gruppierung 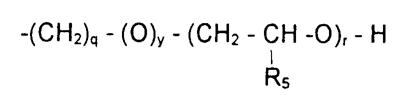 in der bedeuten:
- q eine ganze Zahl zwischen 1 und 5;
- y die ganze Zahl 0 oder 1;
- R₅ ein Wasserstoffatom oder einen Methylrest;
- r eine ganze Zahl zwischen 0 und 10, wobei r und y nicht gleichzeitig 0 sein können;
-(CH₂)ₛ - (CHOH)ₜ - (CH₂)ᵤ - CH₃
in der bedeuten:
- s eine ganze Zahl zwischen 0 und 2;
- t eine ganze Zahl zwischen 0 und 6;
- u eine ganze Zahl zwischen 0 und 5;
-(CH₂)ᵥ - (CHOH)_{w} - CH₂OH
in der bedeuten:
- v die ganze Zahl 0 oder 1;
- w eine ganze Zahl zwischen 1 und 6;
R₄ ein Wasserstoffatom oder die gleiche Bedeutung wie R₃ hat; und
X ein Gegenion, vorzugsweise ein Chloratom, ein Bromatom oder eine Hydroxylgruppe.

2. Quaternäre Ammonium-Derivate der Formel (I) nach Anspruch 1, in der m, R₁, R₂, p, R₃ und R₄ wie in Anspruch 1 definiert sind und R steht für einen linearen oder verzweigten Alkyl- oder Alkenylrest mit 7 bis 17 Kohlenstoffatomen, der vorzugsweise ausgewählt wird aus der Gruppe, welche die folgenden Reste umfaßt:
CH₃-(CH₂)₆-; CH₃-(CH₂)₈-; CH₃-(CH₂)₁₀-; CH₃-(CH₂)₁₂-; CH₃-(CH₂)₁₄-; CH₃-(CH₂)₁₆-; CH₃-(CH₂)₇-CH=CH-(CH₂)₇-; CH₃-(CH₂)₅-CH=CH-(CH₂)₇-; CH₃-(CH₂)₅-CHOH-CH₂-CH=CH-(CH₂)₇-; CH₃-(CH₂)₄-CH=CH-CH₂-CH=CH-(CH₂)₇-; CH₃-(CH₂CH=CH₃)-(CH₂)₇-; H₂C=CH-(CH₂)₈.

3. Quaternäre Ammonium-Derivate der Formel (I) nach Anspruch 2, in der R₃ für einen Rest steht, der ausgewählt wird aus der Gruppe, die besteht aus den Hydroxy-2-ethyl-, Hydroxy-2-propyl-, Hydroxy-3-propyl-, Methyl- und Pentahydroxy-2,3,4,5,6-hexyl-Resten.

4. Quaternäre Ammonium-Derivate der Formel (I) nach Anspruch 1, in der bedeuten:
m die Zahl 3;
R₁ und R₂ einen Methylrest;
p die Zahl 1;
R₃ einen Hydroxy-2-ethylrest;
R₄ ein Wasserstoffatom;
X ein Chloratom oder eine Hydroxylgruppe und
R wie in Anspruch 1 definiert ist.

5. Quaternäre Ammonium-Derivate der Formel (I) nach Anspruch 1, in der bedeuten:
m die Zahl 3;
R₁ und R₂ einen Methylrest;
p die Zahl 1;
R₃ einen Hydroxy-2-ethylrest;
R₄ einen Hydroxy-2-ethylrest;
X ein Chloratom oder eine Hydroxylgruppe; und
R wie in Anspruch 1 definiert ist.

6. Verfahren zur Herstellung der quaternären Ammonium-Derivate der Formel (I) nach einem der Ansprüche 1 bis 5, dadurch gekennzeichnet, daß es umfaßt die Amidierung eines Amins der Formel 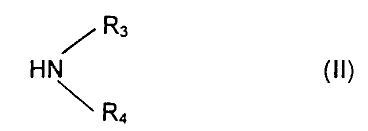 mit einer Verbindung der Formel 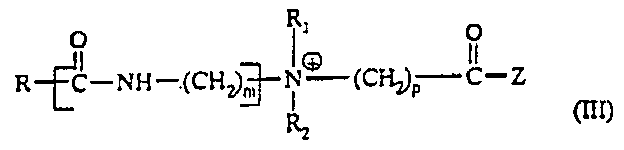 worin R, m, R₁, R₂ und p die gleichen Bedeutungen wie in Anspruch 1 haben und Z für eine Gruppierung OH, OM, OCH₃, OC₂H₅, OC(O)CH₂Cl, Cl steht, worin M ein Alkalimetall darstellt.

7. Verfahren nach Anspruch 6, dadurch gekennzeichnet, daß die obengenannte Verbindung der Formel (III) erhalten wird durch Quaternisierung eines Amins der Formel 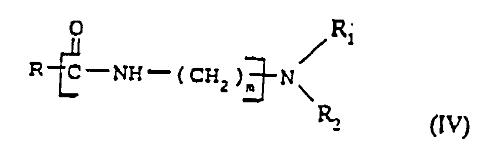 in der R, m, R₁ und R₂ wie in Anspruch 1 definiert sind, mit einer Verbindung der Formel
Cl-CH₂ - CO - Z (V)
in der Z die gleiche Bedeutung wie in Anspruch 7 angegeben hat.

8. Verfahren nach Anspruch 6 oder 7, dadurch gekennzeichnet, daß die Verbindung der Formel (IV) erhalten wird durch Kondensation eines Aminoalkylamins der Formel: 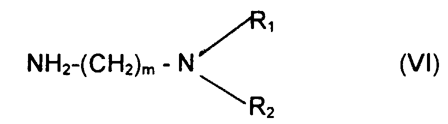 in der m, R₁ und R₂ wie in Anspruch 1 definiert sind,
mit einer Fettsäure der Formel RCOOH oder einem ihrer Vorläufer, insbesondere ihrer Methylsäure oder einem ihrer Glyceride, worin R wie in Anspruch 1 definiert ist.

9. Verfahren nach einem der Ansprüche 6 bis 8, dadurch gekennzeichnet, daß zur Verbesserung der Farbe des obengenannten Produkts der Formel (I) auf die Amidierung des Amins der Formel (II) mit einem quaternären Ammonium-Derivat der Formel (III) eine Behandlung mit einer Verbindung folgt, die ausgewählt wird aus der Gruppe, die Essigsäureanhydrid und Essigsäurechlorid umfaßt, in der Weise, daß die Menge an restlichem Amin auf einen Wert von unter 2 %, vorzugsweise von unter 1 %, reduziert wird.

10. Schaum-, Reinigungs-, Emulgier- oder Konditionier-Zusammensetzungen, dadurch gekennzeichnet, daß sie mindestens eine Verbindung der Formel (I), wie sie in einem der Ansprüche 1 bis 5 definiert ist, in Kombination mit einem anionischen Tensid (oberflächenaktiven Agens) in einem Gewichtsverhältnis zwischen 99:1 und 1:99 umfassen.

11. Verwendung der quaternären Ammonium-Derivate nach einem der Ansprüche 1 bis 5 zur Herstellung von Schaum-, Reinigungs-, Emulgier- und Konditionier-Zusammensetzungen.

## Claims

1. A quaternary ammonium derivative, useful especially as a cationic surfactant. characterised in that it is of the formula: 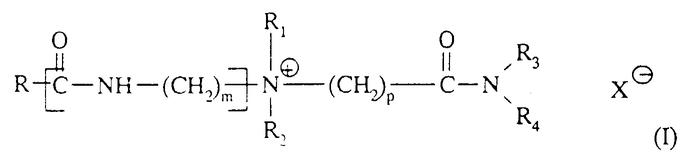 in which:
R represents a linear or branched alkyl or alkenyl radical having 5 to 29 carbon atoms;
m is an integer equal to 2 or 3;
R₁ and R₂ independently represent:
- an alkyl radical having from 1 to 4 carbon atoms, or
- a hydroxymethyl, hydroxyethyl or hydroxypropyl radical;
p is an integer between 1 and 3;
R₃ represents a group: 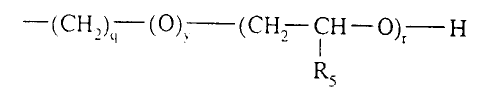 in which:
• q is an integer between 1 and 5,
• y is an integer equal to 0 or 1,
• R₅ represents a hydrogen atom or a methyl radical, and
• r is an integer between 0 and 10. it being specified that r and y cannot simultaneously be equal to 0:
a group
-(CH₂)ₛ-(CHOH)ₜ-(CH₂)ᵤ-CH₃
in which:
• s is an integer between 0 and 2,
• t is an integer between 0 and 6, and
• u is an integer between 0 and 5;
or a group
-(CH₂)ᵥ-(CHOH)_{w}-CH₂OH
in which:
• v is an integer equal to 0 or 1, and
• w is an integer between 1 and 6;
R₄ represents a hydrogen atom or has the same meaning as R₃; and
X represents a counterion. preferably a chlorine or bromine atom or a hydroxyl group.

2. A quaternary ammonium derivative of formula I according to claim 1, in which:
R represents a linear or branched alkyl or alkenyl radical having 7 to 17 carbon atoms, preferably selected from the group comprising the following radicals:
CH₃(CH₂)₆-; CH₃(CH₂)₈-; CH₃(CH₂)₁₀-; CH₃(CH₂)₁₂-; CH₃(CH₂)₁₄-; CH₃(CH₂)₁₆-; CH₃(CH₂)₇-CH=CH-(CH₂)₇-; CH₃(CH₂)₅-CH=CH-(CH₂)₇-; CH₃(CH₂)₅-CHOH-CH₂-CH=CH-(CH₂)₇-; CH₃(CH₂)₄-CH=CH-CH₂-CH=CH-(CH₂)₇-; CH₃(CH₂-CH=CH)₃-(CH₂)₇-; CH₂=CH-(CH₂)₈-; and
m, R₁, R₂, p, R₃ and R₄ are as defined in claim 1.

3. A quaternary ammonium derivative of formula I according to claim 2, in which R₃ represents a radical selected from the group consisting of 2-hydroxyethyl, 2-hydroxypropyl, 3-hydroxypropyl, methyl and 2,3,4,5,6-pentahydroxyhexyl radicals.

4. A quaternary ammonium derivative of formula I according to claim 1 in which:
m is equal to 3;
R₁ and R₂ represent a methyl radical;
p is equal to 1;
R₃ represents a 2-hydroxyethyl radical:
R₄ represents a hydrogen atom;
X represents a chlorine atom or a hydroxyl group; and
R is as defined in claim 1.

5. A quaternary ammonium derivative of formula I according to claim 1 in which:
m is equal to 3;
R₁ and R₂ represent a methyl radical;
p is equal to 1;
R₃ represents a 2-hydroxyethyl radical;
R₄ represents a 2-hydroxyethyl radical;
X represents a chlorine atom or a hydroxyl group; and
R is as defined in claim 1.

6. A process for the preparation of the quaternary ammonium derivatives of formula I according to any one of claims 1 to 5, characterised in that it comprises the amidation of an amine of the formula: 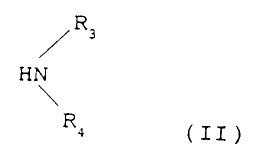 with a compound of the formula: 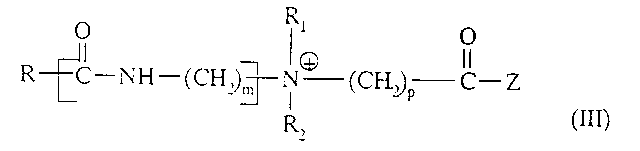 in which R, m, R₁, R₂, p have the same meaning as in claim 1; and
Z represents an OH, OM, OCH₃, OC₂H₅, OC(O)CH₂Cl or Cl group, where M represents an alkali metal.

7. A process according to claim 6, characterised in that the compound of formula (III) given above is obtained by the quaternization of an amine of formula: 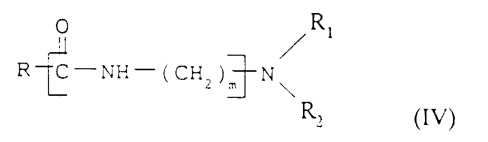 in which R, m, R₁ and R₂ are as defined in claim 1,
with a compound of the formula:
Cl-CH₂-CO-Z (V)
in which Z has the same signification as indicated in claim 9.

8. A process according to claim 6 or 7, characterised in that the compound of formula (IV) is obtained by condensation of an aminoalkylamine of formula: 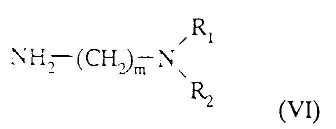 in which m, R₁ and R₂ are as defined in claim 1,
with a fatty acid of the formula RCOOH or a precursor thereof, such as its methyl acid or one of its glycerides, R being as defined in claim 1.

9. A process according to any one of claims 6 to 8, characterised in that in order to improve the colour of the abovementioned product of formula (I), the amidation of the amine of formula (II) with the quaternary ammonium derivative of formula (III) is followed by a treatment with a compound selected from the group comprising acetic anhydride and acetyl chloride, so as to reduce the amount of residual amine to a value below 2%, preferably below 1%.

10. A foaming, cleaning, emulsifying or conditioning composition characterised in that it comprises at least one compound of formula (I) as defined in any one of claims 1 to 5, in combination with an anionic surfactant in a weight ratio of between 99:1 and 1:99.

11. Use of the quaternary ammonium derivatives according to any one of claims 1 to 5 for the preparation of foaming, cleaning, emulsifying or conditioning compositions.
